# EUROPEAN PATENT SPECIFICATION

(11) **EP 3 971 186 B1**
(45) Date of publication and mention of the grant of the patent: **02.08.2023**
(21) Application number: 20827588.3
(22) Date of filing: 18.06.2020
(51) Int. Cl.: C07D 209/08, C07D 417/04, C07D 417/14

(54) **PREPARATION METHOD FOR INDOLE OR INDAZOLE COMPOUND**
VERFAHREN ZUR HERSTELLUNG VON INDOL- ODER INDAZOLVERBINDUNGEN
PROCÉDÉ DE PRÉPARATION D'UN COMPOSÉ INDOLE OU INDAZOLE

(30) Priority: 19.06.2019 KR 20190073020
(43) Date of publication of application: 23.03.2022
(73) Proprietor: LG Chem, Ltd., Seoul 07336 (KR)
(72) Inventor: LEE, Sang Dae, Daejeon 34122 (KR); KIM, Sang Hoon, Daejeon 34122 (KR); HWANG, Gyo Hyun, Daejeon 34122 (KR); PARK, Ae Ri, Daejeon 34122 (KR); KIM, Bong Chan, Daejeon 34122 (KR)
(74) Representative: Hoffmann Eitle
(86) International application number: PCT/KR2020/007910
(87) International publication number: WO 2020/256437

(56) References cited:
- WO-A1-2009/025478
- KR-A- 20090 075 638
- KR-A- 20100 092 909
- KR-A- 20130 056 244
- KR-A- 20150 022 707

## Description

### TECHNICAL FIELD

### Cross-reference to Related Applications

This application claims the benefit of Korean Patent Application No. 10-2019-0073020, filed on June 19, 2019, in the Korean Intellectual Property Office.

### Technical Field

The present invention relates to a novel preparation method of a pharmaceutically useful indole or indazole compound.

### BACKGROUND ART

Typical diseases caused by cellular necrosis include ischemic (e.g. myocardial infarction, stroke, renal infarction), neurodegenerative, and inflammatory diseases. Cellular necrosis is an uncontrolled, accidental cell death under morbid circumstances, and studies on discovering and developing necrosis-inhibiting substances are being carried out in order to treat ischemic, neurodegenerative, and inflammatory diseases and elucidate the biological, pathological causes of cellular necrosis.

Indole derivatives have very useful structure from a medical viewpoint for the inhibition of cellular necrosis, and many results of studies on these structures have been reported. Representative examples include, for example, PCT International Publication No. WO2006/112549, which reported that the compounds have the activity for the glucokinase, PCT International Publication No. WO95/07276, which reported that the compounds are useful as anti-tumor agents and inhibitors against the production of cardiovascular system, and PCT International Publication No. WO2004/018428, which reported that the compounds can be used as antibiotics. In addition, there is PCT International Publication No. WO2009/025478 which reported a novel indole or indazole derivative structure useful for an effect of prevention or treatment and amelioration for cellular necrosis and necrosis-associated diseases.

Among these, the present invention relates to a novel method for preparing an indole or indazole compound, which exhibits an effect of prevention or treatment and amelioration for cellular necrosis and necrosis-associated diseases.

Conventionally, borane dimethylsulfide (BH₃SMe₂) was used in the synthesis process of indole or indazole compounds, but borane dimethylsulfide (BH₃SMe₂) is a toxic substance, thus causing a safety problem, and a poor yield problem since the process is performed in a complicated procedure through a very large number of steps.

In addition, a preparation method of reducing an indole or indazole compound containing a nitro group using an iron (Fe) catalyst to an indole or indazole compound containing an amine group has been conventionally used. However, in this case, there have been limitations in that reproducibility is deteriorated due to various particle sizes of iron (Fe), stirring is not smooth during the reaction, or yield is lowered due to impurities generated in the reaction when the reaction time is prolonged. In addition, there has been a limitation in that a reactor is coated with iron oxide produced after the reaction and the cost and time is required to clean the reactor.

Therefore, there is a need to develop a new preparation method which solves limitations occurring in the conventional preparation process and improves yield and the like.

### [PRIOR ART DOCUMENTS]

### [PATENT DOCUMENTS]

PCT International Publication WO2006/112549 (October 26, 2006)
PCT International Publication WO1995/007276 (March 16, 1995)
PCT International Publication WO2004/018428 (March 4, 2004)
PCT International Publication WO2009/025478 (February 26, 2009)

### DISCLOSURE OF THE INVENTION

### TECHNICAL PROBLEM

An aspect of the present invention provides a novel method for preparing a pharmaceutically useful indole or indazole compound, which can ensure safety, improve yield through the simplification of the process, and solve the limitations of the conventional method using an iron (Fe) catalyst.

### TECHNICAL SOLUTION

According to an aspect of the present invention, there is provided a method for preparing a compound represented by Formula 2 below by including a compound represented by Formula 1 below as a reaction intermediate: 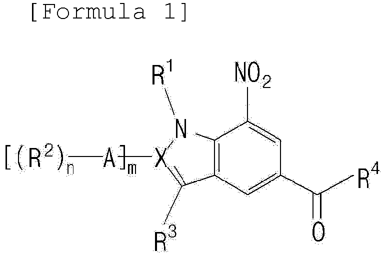 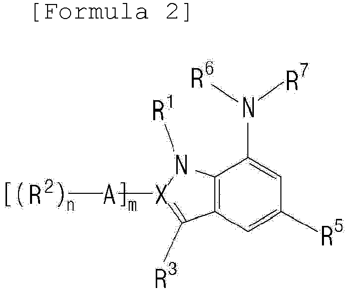
wherein, in Formulae above,
n is an integer of 1 to 3,
m is 0 or 1,
A represents phenyl, or represents 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
X represents C or N, with the proviso that m is 0 when X is N and m is 1 when X is C,
R¹ represents hydrogen, C₁-C₆-alkyl or - (CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents -(CH₂)ₚCO₂R⁸, -(CH₂)ₚOR⁸, -(CH₂)ₚNR⁸R⁹, -NHR¹⁰,-N(H)S(O)₂R⁸, or -NHC(O)R¹⁰, or represents -(CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom,
R³ represents hydrogen, halogen, C₁-C₆-alkyl or phenyl, or represents -(CH2)ₛ-heterocycle in which the heterocycle is a 5-6 membered ring containing one or two heteroatoms selected from among N and O atoms, wherein s is an integer of 1 to 3, with the proviso that R³ is hydrogen or phenyl when X is N,
R⁴ represents -YR¹¹, wherein Y is a direct bond or represents -(CR⁸R⁹)_{g}-, wherein g is an integer of 0 to 3, R⁸ and R⁹ are the same as defined above, R¹¹ is selected from the group consisting of hydrogen, halogen, C₁-C₆-alkyl and - (CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ represents hydrogen or C₁-C₆-alkyl when X is N,
R⁵ represents -Y'R¹¹, wherein Y' is a direct bond or represents -(CR⁸R⁹)ₕY"-, wherein h is an integer of 0 to 3, and R⁸, R⁹, and R¹¹ are the same as defined above,
Y" is selected from the group consisting of -O-,-C(O)-, and -C(O)O-, with the proviso that R⁵ represents hydrogen or C₁-C₆-alkyl when X is N,
R⁶ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁶ is hydrogen when X is N, and
R⁷ represents - (CR⁸R⁹)ᵤ-Z-D-W-R¹⁴, wherein u is an integer of 0 to 3, Z represents a direct bond or is selected from the group consisting of -C(O)- and -C(0)0-, D represents a direct bond, C₄-C₆-cycloalkyl, or a 5-6 membered heteroaryl containing one or two N atoms, or represents a 5-6 membered heterocycle containing one or two heteroatoms selected from among N, O and S atoms, W represents a direct bond, or -NR⁸-, -C(O)-, -C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, with the proviso that when X is N, R⁷ represents C₄-C₆-cycloalkyl, or a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

### ADVANTAGEOUS EFFECTS

According to the present invention, safety can be ensured due to the non-use of borane dimethylsulfide (BH₃SMe₂), yield can be improved through the simplification of the process, an indole or indazole compound can be prepared in excellent yield with reproducibility by solving limitations of a conventional iron (Fe) catalyst, and the costs and time required for cleaning the reactor can be reduced by solving the limitation of iron oxide coating of the reactor.

### MODE FOR CARRYING OUT THE INVENTION

Hereinafter, the present invention will be described in more detail with reference to the accompanying drawings. In this case, terms or words used in the specification and claims should not be interpreted as being limited to a conventional or dictionary meaning, and should be interpreted as the meaning and concept that accord with the technical spirit on the grounds of the principle that the inventor can appropriately define the concept of the term in order to explain the invention in the best way.

In the definition for the substituents of Formulae according to the present invention, the term "alkyl" refers to an aliphatic hydrocarbon radical. Alkyl may be "saturated alkyl" which does not include an alkenyl or alkynyl moiety, or "unsaturated alkyl" which includes at least one alkenyl or alkynyl moiety. The term "alkenyl" refers to a group containing at least one carbon-carbon double bond, and the term "alkynyl" refers to a group containing at least one carbon-carbon triple bond Alkyl may be branched or linear when used alone or in a composite form such as alkoxy.

The alkyl group may have 1 to 20 carbon atoms unless otherwise defined. The alkyl group may be medium-sized alkyl having 1 to 10 carbon atoms. The alkyl group may be lower alkyl having 1 to 6 carbon atoms. A typical alkyl group includes, but is not limited to, methyl, ethyl, propyl, isopropyl, butyl, isobutyl, t-butyl, pentyl, hexyl, ethenyl, propenyl, butenyl, etc. For example, C₁-C₄-alkyl has 1 to 4 carbon atoms in the alkyl chain, and is selected from the group consisting of methyl, ethyl, propyl, iso-propyl, n-butyl, iso-butyl, sec-butyl and t-butyl.

The term "alkoxy" refers to alkyloxy having 1 to 10 carbon atoms unless otherwise defined.

The term "cycloalkyl" refers to a saturated aliphatic 3-10 membered ring unless otherwise defined. A typical cycloalkyl group includes, but is not limited to, cyclopropyl, cyclobutyl, cyclopentyl, cyclohexyl, etc.

The term "aryl" includes at least one ring having a covalent π electron system, for example, a monocyclic or fused polycyclic (i.e., rings that share adjacent pairs of carbon atoms) group. That is, unless otherwise defined herein, aryl refers to an aromatic 4-10 membered, preferably 6-10 membered, monocyclic or multicyclic ring including phenyl, naphthyl, etc.

The term "heteroaryl" refers to an aromatic 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl unless otherwise defined. Examples of monocyclic heteroaryl include, but are not limited to, thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine and the like. Examples of bicyclic heteroaryl include, but are not limited to, indole, indoline, benzothiophene, benzofuran, benzimidazole, benzoxazole, benzisoxazole, benzthiazole, benzthiadiazole, benztriazole, quinoline, isoquinoline, purine, furopyridine and the like.

The term "heterocycle" refers to a 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl, and is saturated or contains 1 or 2 double bonds, unless otherwise defined. Examples of the heterocycle include, but are not limited to, pyrroline, pyrrolidine, imidazoline, imidazolidine, pyrazoline, pyrazolidine, pyran, piperidine, morpholine, thiomorpholine, piperazine, hydrofuran, and the like.

Unless otherwise defined, terms and abbreviations used herein may be interpreted as meanings commonly understood by those skilled in the art to which the present invention pertains.

The present invention relates to a method for preparing a pharmaceutically useful indole or indazole compound, and provides the method for preparing a compound represented by Formula 2 below by including a compound represented by Formula 1 below as a reaction intermediate: 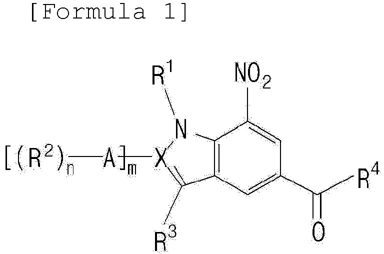
wherein, in Formula 1 above,
n is an integer of 1 to 3,
m is 0 or 1,
A represents phenyl, or represents 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
X represents C or N, with the proviso that m is 0 when X is N and m is 1 when X is C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents -(CH₂)ₚCO₂R⁸, -(CH₂)ₚOR⁸, -(CH₂)ₚNR⁸R⁹, -NHR¹⁰,-N(H)S(O)₂R⁸, or -NHC(O)R¹⁰, or represents -(CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom,
R³ represents hydrogen, halogen, C₁-C₆-alkyl or phenyl, or represents -(CH₂)ₛ-heterocycle in which the heterocycle is a 5-6 membered ring containing one or two heteroatoms selected from among N and O atoms, wherein s is an integer of 1 to 3, with the proviso that R³ is hydrogen or phenyl when X is N,
R⁴ represents -YR¹¹, wherein Y is a direct bond or represents -(CR⁸R⁹)_{g}-, wherein g is an integer of 0 to 3, R⁸ and R⁹ are the same as defined above, R¹¹ is selected from the group consisting of hydrogen, halogen, C₁-C₆-alkyl and - (CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ represents hydrogen or C₁-C₆-alkyl when X is N.

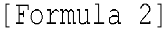

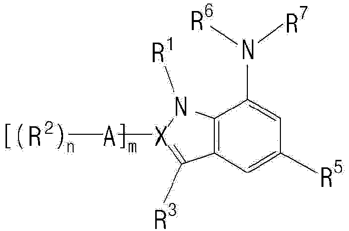

In Formula 2 above, n, m, X, A, R¹, R², and R³ are the same as defined above,
R⁵ represents -Y'R¹¹, wherein Y' is a direct bond or represents -(CR⁸R⁹)ₕY"-, wherein h is an integer of 0 to 3, and R⁸, R⁹, and R¹¹ are the same as defined above,
Y" is selected from the group consisting of -O-, - C(O)-, and -C(O)O-, with the proviso that R⁵ represents hydrogen or C₁-C₆-alkyl when X is N,
R⁶ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁶ is hydrogen when X is N, and
R⁷ represents -(CR⁸R⁹)ᵤ-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, or represents a 5-6 membered heteroaryl containing one or two N atoms, or represents a 5-6 membered heterocycle containing one or two heteroatoms selected from among N, O and S atoms, W represents a direct bond, or -NR⁸-,-C(O)-, -C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, with the proviso that when X is N, R⁷ represents C₄-C₆-cycloalkyl, or a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

Specifically, in Formula 1 and Formula 2 above, R¹ above may be hydrogen, C₁-C₆-alkyl, or di(C₁-C₃-alkyl) amino-C₂-C₃-alkyl.

In addition, R² above may represent hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, -(CH₂)ₚNR⁸R⁹,-NHR¹⁰, -N(H)S(O)₂R¹⁰ or -NHC(O)₂R¹⁰ or may be -(CH₂)ₚ-heterocycle-R¹⁰, wherein heterocycle, p, R⁸, R⁹ and R¹⁰ are as defined above.

Further, R³ may represent hydrogen, methyl or halogen, or represent phenyl optionally substituted by C₁-C₃-alkoxy, or may be heterocyclyl-C₁-C₃-alkylene in which the heterocycle is a 5-6 membered ring containing one or two heteroatoms selected from N and O atoms and optionally substituted with one or two oxo groups.

In addition, Y above may be a direct bond, and Y' above may be a direct bond, or may be selected from the group consisting of a direct bond, -O-, -C(O)-, and -CH₂C(O)-.

In addition, R¹¹ above may be selected from the group consisting of hydrogen, methyl, ethyl, phenyl, fluoro, chloro, 2-carboxy-pyrrolidin-1-yl, pyrrolidin-1-yl, 4-acetic acid-1,3-thiazolin-2-yl, -CH₂-(1,1-dioxo-thiomorpholin-4-yl),-CH₂-(2-oxopiperazin-4-yl), -1,1-dioxo-thiomorpholin-4-yl, and -2-oxopiperazin-4-yl.

Also, in Formula 2 above, R⁶ above may be hydrogen, methyl, or isobutyl.

R⁷ above may be selected from the group consisting of cyclobutyl, cyclopentyl, cyclohexyl, 4-methyl-cyclopentyl, 4,4-difluorocyclohexyl, or D may be selected from the group consisting of cyclopentyl, cyclohexyl, pyrrolidine, tetrahydropyran, tetrahydrofuran and piperidine, and W may represent a direct bond or may represent -SO₂-, -CO-, -C(O)O- or -CONR¹²-, wherein R¹² is the same as defined above.

As an embodiment of the present invention, the compound represented by Formula 1 above may be (1,1-dioxydothiomorpholino)-(7-nitro-2-phenyl-1H-indol-5-yl)methanone, and the compound represented by Formula 2 above may be 4-((2-phenyl-7-((tetrahydro-2H-pyran-4-yl)amino)-1H-indol-5-yl)methyl)thiomorpholine 1,1-dioxide.

The compound represented by Formula 1 above may be prepared by amidating a compound represented by Formula 3 below:

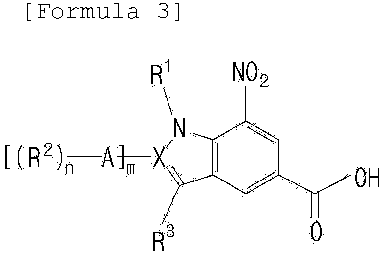

In Formula 3 above, n, m, X, A, R¹, R², and R³ are the same as defined above,

The amidating step is not specifically limited, and may be performed by a conventional manner.

Conventionally, a step for reducing the compound represented by Formula 3 above was performed using borane dimethylsulfide (BH₃SMe₂). There was a safety issue since borane dimethylsulfide (BH₃SMe₂) used in this step is a toxic substance. However, according to the present invention, the step for amidating the compound represented by Formula 3 above to prepare an intermediate represented by Formula 1 above is performed, thereby ensuring safety due to the non-use of borane dimethylsulfide (BH₃SMe₂) .

Next, the present invention may include a step for performing a primary reduction and a secondary reduction of the compound represented by Formula 1 above to prepare a compound represented by Formula 4 below:

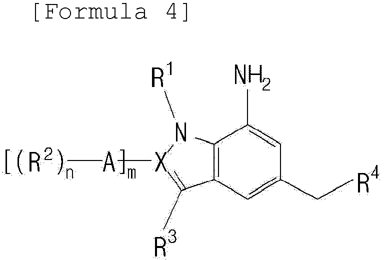

In Formula 4 above, n, m, X, A, R¹, R², R³ and R⁴ are the same as defined above,

In this case, the primary reduction may be performed by using, as a reducing agent, at least one selected from the group consisting of sodium borohydride (NaBH₄), boron trifluoride etherate (BF₃OEt₂), and lithium aluminum hydride (LAH), and more preferably, sodium borohydride (NaBH₄) and boron trifluoride etherate (BF₃OEt₂) may be used together. The primary reduction reduces the carbonyl group (C=O) of the compound represented by Formula 1 to produce a compound represented by Formula 5 below:

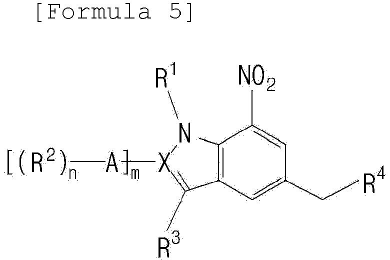

In Formula 5 above, n, m, X, A, R¹, R², R³ and R⁴ are the same as defined above,

The secondary reduction may be performed by using, as a reducing agent, at least one selected from the group consisting of Pd/C, NiBr₂, NiCl₂, ZnCl₂, ZrCl₄, I₂, BiCl₃, Cu, FeCl₃·6H₂O, In(OTf₃), Ni, NiCl₂·6H₂O, SnCl₂, LiBH₄, LiCl, NaBF₄, and CoCl₂·6H₂O, and more preferably, Pd/C. Through the secondary reduction, the nitro group (-NO₂) of the compound represented by Formula 5 above may be reduced to the amine group (-NH₂) of the compound represented by Formula 4.

Conventionally, an iron (Fe) catalyst was used in the step for reducing the compound represented by Formula 5 which contains the nitro group (-NO₂) to the compound represented by Formula 4 which contains the amine group (-NH₂) during the synthesis of indole or indazole compounds. However, in this case, there have been limitations in that reproducibility is deteriorated due to various particle sizes of iron (Fe), stirring is not smooth during the reaction, or yield is lowered due to impurities generated in the reaction when the reaction time is prolonged. In addition, there has been a limitation in that a reactor is coated with iron oxide produced after the reaction and the cost and time is required to clean the reactor. However, the present invention may prepare the indole or indazole compound in excellent yield and have reproducibility by solving the limitations of the conventional iron (Fe) catalyst by performing the primary and secondary reduction as in the above manner without using the iron (Fe) catalyst, and the costs and time required for cleaning the reactor may be reduced by solving the limitation of iron oxide coating of the reactor.

In addition, conventionally, in the synthesis of indole or indazole compounds, when the compound represented by Formula 5 above was prepared from the compound represented by Formula 3 above, the compound represented by Formula 3 above was reduced by employing borane dimethylsulfide (BH₃SMe₂) (Step 1), halogenated (Step 2), and subjected to amine substitution again (Step 3). However, when the compound represented by Formula 5 above is prepared from the compound represented by Formula 3 above, the present invention may be carried out in a total of two steps, namely, a step (Step 1) for amidating the compound represented by Formula 3 and a step (Step 2) for reducing a carbonyl group (C=O) through a primary reduction. Thus, the present invention improves synthesis efficiency and yield through the simplification of the process.

The preparation method of the present invention may comprise a step for reacting the compound represented by Formula 4 above with ketone or aldehyde to prepare the compound represented by Formula 2 above.

Hereinafter, embodiments of the present invention will be described in detail so that a person with ordinary skill in the art can easily practice the present invention. The present invention may, however, be embodied in different forms and should not be construed as limited to the embodiments set forth herein.

### Example

### Step a) Synthesis of (1,1-dioxydothiomorpholino)(7-nitro-2-phenyl-1H-indol-5-yl)methanone

7-nitro-2-phenyl-1H-indole-5-carboxylic acid (250 g), HOBt·H₂O [C₆H₅N₃O·H₂O] (54.25 g), 1-(3-dimethylaminopropyl)-3-ethylcarbodiimide·hydrochloride (EDC·HCl) (186.78 g), 1,1-dioxothiomorpholine·hydrochloride (151.98 g), diisopropylethylamine (DIPEA) (154.27 mL), and N,N-dimethylformamide (DMF) (1.37 L) were added to a reactor and then stirred at room temperature. The reaction mixture was heated to 75-80 °C and the reaction was terminated when the reaction mixture was analyzed by sampling since 1 hour after the heating to show 1.0% or less of the peak of 7-nitro-2-phenyl-1H-indole-5-carboxylic acid.

After confirming the completion of the reaction, the reaction mixture was cooled to 35-40 °C, H₂O (3.75 L) was slowly added dropwise and then stirred at room temperature for 1 hour. The reaction mixture was stirred, filtered, and washed with H₂O (0.75 L), and then washed with ethanol/H₂O (1/2, 0.75 L) again. The reaction mixture was dried under N₂ pressure to synthesize crude (1,1-dioxydothiomorpholino) (7-nitro-2-phenyl-1H-indol-5-yl)methanone (336.09 g, purity: 93.43%). The crude (1,1-dioxydothiomorpholino) (7-nitro-2-phenyl-1H-indol-5-yl)methanone (336.09 g) and tetrahydrofuran (THF, 2.69 L) were added thereto at room temperature, then the temperature was raised, and the reaction mixture was refluxed and stirred for 1 hour. The reaction mixture was cooled to 35-40 °C, H₂O (4.03 L) was added dropwise thereto, and stirred at room temperature for 1 hour and washed with THF/H₂O (1 L). The resultant was dried under N₂ pressure to synthesize (1,1-dioxydothiomorpholino) (7-nitro-2-phenyl-1H-indol-5-yl)methanone (314.1 g, yield: 88.78%, purity: 95.87%).

¹H NMR (400MHz, DMSO-d₆) δ 11.87 (s, 1H), 8.25 (s, 1H), 8.19 (s, 1H), 8.02 (m, 2H), 7.52 (t, J= 7.6 Hz, 2H) 7.43 (dd, J= 6.8, 7.2 Hz, 1H), 7.23 (s, 1H), 3.93 (m, 4H), 3.30 (m, 4H) .

### Step b) Synthesis of 4-((7-amino-2-phenyl-1H-indole-5-yl)methyl)thiomorpholine 1,1-dioxide

NaBH₄ (1.137 g) and THF (30 ml) were added to a reactor, the reactor was cooled to a temperature of 10 °C or less, and then boron trifluoride etherate (BF₃OEt₂) (3.71 mL) was slowly added thereto. (1,1-dioxydothiomorpholino) (7-nitro-2-phenyl-1H-indol-5-yl)methanone (4 g) was added thereto and heated to 40 °C, and the reaction was terminated when the reaction mixture was analyzed by sampling since 1 hour after the heating to show 1.0% or less of the peak of (1,1-deoxythiomorpholino) (7-nitro-2-phenyl-1H-indol-5-yl)methanone.

The reactor was cooled to a temperature of 10 °C, 10 wt% Pd/C (53 mg) and NaBH₄ (1.137 g) were added thereto, and then ethanol (40 ml) was added dropwise at the same temperature. The reaction was terminated when the reaction mixture was analyzed by sampling since 0.5 hour after the addition of ethanol to show 1.0% or less of the peak of 4-((7-nitro-2-phenyl-1H-indol-5-yl)methyl)thiomorpholine 1,1-dioxide. The temperature of the reaction solution was raised to 25-30 °C, then H₂O (20 ml) was added thereto, and the reaction mixture was stirred for 30 minutes and concentrated under reduced pressure. H₂O (80 mL) was added thereto and heated to 80-90 °C, and then stirred for 1 hour and filtered. The reaction mixture was primarily washed with H₂O (20 mL) and secondarily washed with ethanol/H₂O (1/2, 20 mL), and then dried under N₂ pressure to synthesize crude 4-((7-amino-2-phenyl-1H-indol-5-yl)methyl)thiomorpholine 1,1-dioxide (3.51 g, purity: 98.42%).

The crude 4-((7-amino-2-phenyl-1H-indol-5-yl)methyl)thiomorpholine 1,1-dioxide (3.51 g) and H₂O (52 mL) were added to a reactor, cooled to 10 °C or less, and methanesulfonic acid (1.8 mL) was then added dropwise to adjust the pH to 3-4 while maintaining the temperature at or below 25 °C. The reaction mixture was stirred at room temperature for 30 minutes, filtered with celite, and washed with H₂O (11 mL). 6N NaOH (5 mL) was added to the filtrate to be a pH of 9, and then stirred at room temperature for 30 minutes and filtered. The resultant was primarily washed with H₂O (11 mL) and secondarily washed with ethanol/H₂O (1/2, 11 mL), and then dried under N₂ pressure to synthesize 4-((7-amino-2-phenyl-1H-indol-5-yl)methyl)thiomorpholine 1,1-dioxide (3.13 g, yield: 87.92%, purity: 91.31%).

¹H NMR (500MHz, DMSO-d₆) δ 10.89 (s, 1H), 7.80 (m, 2H), 7.48 (t, J= 7.7 Hz, 2H) 7.30 (d, J= 7.3 Hz, 1H), 6.74 (m, 2H), 6.34 (s, 1H), 5.15 (s, 2H), 3.07 (m, 4H), 2.87 (m, 4H).

### Step c) Synthesis of 4-((2-phenyl-7-((tetrahydro-2H-pyran-4-yl)amino)-1H-indol-5-yl)methyl)thiomorpholine 1,1-dioxide

4-((7-amino-2-phenyl-1H-indol-5-yl)methyl)thiomorpholine 1,1-dioxide (3.8 kg), tetrahydro-4H-pyran-4-one (1.3 kg), acetic acid (AcOH) (4.6 kg), and isopropyl acetate (23.2 L) were added to a reactor and stirred, and then NaBH(OAc)₃ (4.6 kg) was added thereto, followed by stirring at room temperature for 1 hour or more, and the reaction was terminated when the reaction mixture was analyzed to show 0.5% or less of the peak of 4-((7-amino-2-phenyl-1H-indol-5-yl)methyl)thiomorpholine 1,1-dioxide.

The completion of the reaction was confirmed, a 1N NaOH aqueous solution (23.2 L) was added to the reaction mixture, followed by stirring for another 1 hour or more, and then the organic solvent was distilled under reduced pressure. The solid present in the remaining aqueous solution was filtered, washed with water (38.7 L) and t-butyl methyl ether (38.7 L), and dried under N₂ pressure for 16 hours to synthesize 4-((2-phenyl-7-((tetrahydro-2H-pyran-4-yl)amino)-1H-indole-5-yl)methyl)thiomorpholine 1,1-dioxide (3.9 kg, yield: 82.4%, purity: 98.5%).

¹H NMR (500MHz, DMSO-d₆) δ 10.96 (s, 1H), 7.80 (m, 2H), 7.48 (t, J= 7.7 Hz, 2H) 7.30 (d, J= 7.3 Hz, 1H), 6.74 (s, 2H), 6.30 (s, 1H), 5.36 (m, 1H), 3.95 (m, 2H), 3.62 (s, 2H), 3.50 (m, 2H), 3.09 (m, 4H), 2.87 (m, 4H), 2.05 (m, 2H), 1.45 (m, 2H).

### Comparative Example

### Step a) Synthesis of (7-nitro-2-phenyl-1H-indol-5-yl) methanol

7-nitro-2-phenyl-1H-indole-5-carboxylic acid (5.8 kg) and THF (61 L) were added to a reactor at room temperature and stirred, and then the reaction mixture was cooled to 10-15 °C, and 5M borane dimethylsulfide (BH₃SMe₂, 12.3 L) was slowly added dropwise thereto so that the internal temperature thereof did not exceed 40 °C. After the completion of the dropwise addition, the reactor was heated to a temperature of 40 °C, the reaction mixture was stirred for 2 hours, and then the reaction was terminated when the reaction mixture was analyzed by HPLC to show 1.0% or less of the peak of 7-nitro-2-phenyl-1H-indole-5-carboxylic acid.

After confirming the completion of the reaction, H₂O (55 L) was slowly added dropwise to the reaction mixture, and then the mixture was separated into layers to store an organic layer, and the water layer was extracted with ethyl acetate (EtOAc, 29 L), mixed with the stored organic layer, and distilled under reduced pressure. EtOH/H₂O (6.1 L/12.2 L) was added to the obtained concentrate, stirred for at least 2 hours, and then filtered. The resultant was washed with a washing solution (EtOH/H₂O = 1/3, 9.0 L) and then dried under N₂ pressure for 16 hours to synthesize (7-nitro-2-phenyl-1H-indol-5-yl)methanol (4.6 kg, yield: 83.5%, purity: 98.4%).

¹H NMR (500MHz, DMSO-d₆) δ 11.6 (s, 1H), 8.07 (s, 1H), 8.00 (m, 3H), 7.49 (t, J= 7.7 Hz, 2H) 7.40 (d, J= 7.3 Hz, 1H), 7.14 (s, 1H), 5.42 (t, J= 6.7 Hz, 1H), 4.66 (d, J= 6.7 Hz, 2H) .

### Step b) Synthesis of 4-((7-nitro-2-phenyl-1H-indol-5-yl)methyl)thiomorpholine 1,1-dioxide

(7-nitro-2-phenyl-1H-indole-5-yl)methanol (4.6 kg) and THF (46 L) were added to a reactor and stirred at room temperature, the reactor was cooled to a temperature of 0 °C, and then PBr₃ (2.8 kg) was added while the internal temperature of the reaction was maintained below 20 °C. After the completion of the addition, the temperature of the reactor was raised to 25 °C, the mixture was stirred for 1 hour, and the reaction was terminated when the reaction mixture was analyzed by sampling to show 1.0% or less of the peak of 5-(hydroxymethyl)-7-nitro-2-phenyl-1H-indole.

The completion of the reaction was confirmed, the reaction mixture was cooled to 0 °C, and 1,1-dioxothiomorpholine hydrochloride (4.4 kg) and N,N-diisopropylethylamine (DIPEA) (8.0 kg) were added while the internal temperature of the reaction was maintained below 20 °C. After the completion of the addition, the temperature of the reactor was raised to 50 °C, the mixture was stirred for 4 hours, and the reaction was terminated when the reaction mixture was analyzed by sampling to show 1.0% or less of the peak of 5-(bromomethyl)-7-nitro-2-phenyl-1H-indole.

After confirming the completion of the reaction, the reactor was cooled to a temperature of 0 °C, water (46.0 L) was added thereto, the reaction mixture was stirred, and the organic solvent was distilled under reduced pressure. After the distillation under reduced pressure, ethyl acetate (EtOAc) (48.0 L) was additionally added thereto, and the organic solvent was distilled under reduced pressure again to obtain 4-((7-nitro-2-phenyl-1H-indol-5-yl)methyl)thiomorpholine 1,1-dioxide as a solid, and then filtered. The resultant was washed with water (26.0 L) and ethanol (26.0 L) and dried under N₂ pressure for 16 hours to synthesize crude 4-((7-nitro-2-phenyl-1H-indole-5-yl)methyl)thiomorpholine 1,1-dioxide (6.4 kg, purity: 92.9%).

For purification, crude 4-((7-nitro-2-phenyl-1H-indole-5-yl)methyl)thiomorpholine 1,1-dioxide above was added to a mixture of DMF (6.0 L) and toluene (60.0 L), stirred, heated to 120 °C, stirred for 2 hours, and then stirred for another 2 hours at room temperature again. Toluene (60.0 L) was additionally added thereto and stirred for 2 another hours, and then a solid in the solution was filtered and washed with toluene (20.0 L) to synthesize 4-((7-nitro-2-phenyl-1H-indole-5-yl)methyl)thiomorpholine 1,1-dioxide (5.1 kg, yield: 77.0% overall, purity: 98.4%).

¹H NMR (500MHz, DMSO-d₆) δ 11.63 (s, 1H), 8.07 (s, 1H), 8.04 (m, 3H), 7.49 (t, J= 7.7 Hz, 2H) 7.38 (d, J= 7.3 Hz, 1H), 7.14 (s, 1H), 3.79 (s, 2H), 3.12 (m, 4H), 2.84 (m, 4H).

### Step c) Synthesis of 4-((7-amino-2-phenyl-1H-indol-5-yl)methyl)thiomorpholine 1,1-dioxide

4-((7-nitro-2-phenyl-1H-indole-5-yl)methyl)thiomorpholine 1,1-dioxide (0.60 kg), THF/methanol/H₂O (1:1:1, 3.0 L), and NH₄Cl (124 g) were added to a reactor at room temperature and stirred, and then Fe (480 g) was added thereto. The reactor was heated to a temperature of 60 °C, the reaction mixture was stirred for 1 hour or more, and then the reaction was terminated when the reaction mixture was analyzed to show 0.5% or less of the peak of 4-((7-nitro-2-phenyl-1H-indole-5-yl)methyl)thiomorpholine 1,1-dioxide.

The completion of the reaction was confirmed, THF (3.0 L) was added to the reaction mixture and stirred for 10 minutes, and then filtered with celite, and the resultant was washed with THF/H₂O (1: 1, 3.0 L). The filtrate was under reduced pressure and THF and methanol were distilled to form a solid, and the reaction mixture was stirred at room temperature for 2 hours or more. The resultant was filtered and washed with the washing solution [1st H₂O: 500 mL, 2nd ethanol/H₂O = 1/3, 1.0 L], and then dried under N₂ pressure for 16 hours to synthesize 4-((7-amino-2-phenyl-1H-indol-5-yl)methyl)thiomorpholine 1,1-dioxide [0.45 kg (yield: 81.3 %, purity: 97.2%)].

¹H NMR (500MHz, DMSO-d₆) δ 10.89 (s, 1H), 7.80 (m, 2H), 7.48 (t, J= 7.7 Hz, 2H) 7.30 (d, J= 7.3 Hz, 1H), 6.74 (m, 2H), 6.34 (s, 1H), 5.15 (s, 2H), 3.07 (m, 4H), 2.87 (m, 4H).

Next, 4-((2-phenyl-7-((tetrahydro-2H-pyran-4-yl)amino)-1H-indol-5-yl)methyl)thiomorpholine 1,1-dioxide (3.9 kg, yield: 82.4%, purity: 98.5%) was prepared in the same manner as in Step c) of Example 1.

Referring to the Example and Comparative Example, a total yield of 4-(7-amino-2-phenyl-1H-indol-5-yl)methyl)thiomorpholine 1,1-dioxide was 78.06% (the value in which 88.78% of Step a) and 87.92% of Step b) were multiplied) in the Example in which 4-(7-amino-2-phenyl-1H-indol-5-yl)methyl)thiomorpholine 1,1-dioxide was synthesized from 7-nitro-2-phenyl-1H-indol-5-carboxylic acid through the two steps according to the present invention, and a total yield of 4-(7-amino-2-phenyl-1H-indol-5-yl)methyl)thiomorpholine 1,1-dioxide was calculated to be 52.27% (the value in which 83.5% of Step a), 77.0% of Step b), and 81.3% of Step c) were multiplied) in the Comparative Example in which the compound was synthesized through the three steps. That is, it may be confirmed that the present invention significantly improves yield through the simplification of the process.

## Claims

1. A method for preparing a compound represented by Formula 2 below by comprising a compound represented by Formula 1 below as a reaction intermediate: 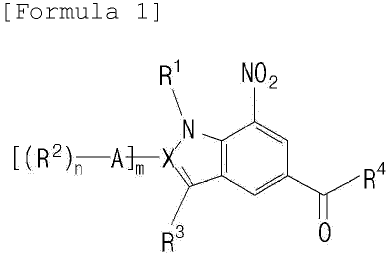 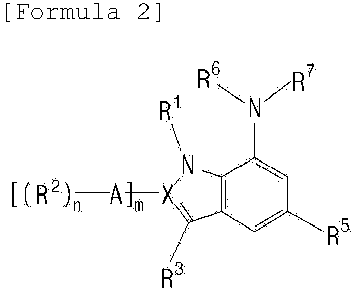
wherein, in Formulae above,
n is an integer of 1 to 3,
m is 0 or 1,
A represents phenyl, or represents 5-membered heteroaryl or heterocycle which each contains 1 to 3 heteroatoms selected from among N, O and S atoms and is optionally substituted by R, wherein R represents hydrogen or C₁-C₄-alkyl optionally substituted by hydroxy or amino,
X represents C or N, with the proviso that m is 0 when X is N and m is 1 when X is C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents -(CH₂)ₚCO₂R⁸, -(CH₂)ₚOR⁸, -(CH₂)ₚNR⁸R⁹, -NHR¹⁰,-N(H)S(O)₂R8, or -NHC(O)R¹⁰, or represents -(CH₂)ₚ-heterocycle-R¹⁰ in which the heterocycle moiety is a 5-6 membered ring containing one or two heteroatoms selected from among N, O, and S atoms, wherein p is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom,
R³ represents hydrogen, halogen, C₁-C₆-alkyl or phenyl, or represents -(CH₂)ₛ-heterocycle in which the heterocycle is a 5-6 membered ring containing one or two heteroatoms selected from among N and O atoms, wherein s is an integer of 1 to 3, with the proviso that R³ is hydrogen or phenyl when X is N,
R⁴ represents -YR¹¹, wherein Y is a direct bond or represents - (CR⁸R⁹)_{g}-, wherein g is an integer of 0 to 3, R⁸ and R⁹ are the same as defined above, R¹¹ is selected from the group consisting of hydrogen, halogen, C₁-C₆-alkyl and - (CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ represents hydrogen or C₁-C₆-alkyl when X is N,
R⁵ represents -Y'R¹¹, wherein Y' is a direct bond or represents -(CR⁸R⁹)ₕY"-, wherein h is an integer of 0 to 3, and R⁸, R⁹, and R¹¹ are the same as defined above,
Y" is selected from the group consisting of -O-, -C(O)-, and -C(O)O-, with the proviso that R⁵ represents hydrogen or C₁-C₆-alkyl when X is N,
R⁶ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁶ is hydrogen when X is N, and
R⁷ represents -(CR⁸R⁹)ᵤ-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, or a 5-6 membered heteroaryl containing one or two N atoms, or represents a 5-6 membered heterocycle containing one or two heteroatoms selected from among N, O and S atoms, W represents a direct bond, or -NR⁸-, -C(O)-, -C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, with the proviso that when X is N, R⁷ represents C₄-C₆-cycloalkyl, or a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

2. The method of claim 1, wherein the compound represented by Formula 1 above is prepared by amidating the compound represented by Formula 3 below: 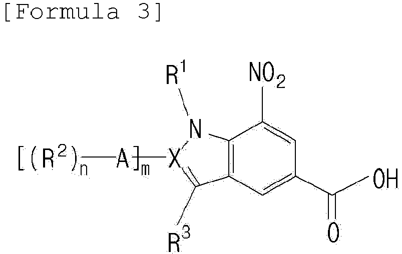 wherein, in Formula above, n, m, X, A, R¹, R², and R³ are the same as defined in claim 1,

3. The method of claim 1, comprising a step for performing a primary reduction and a secondary reduction of the compound represented by Formula 1 above to prepare a compound represented by Formula 4 below: 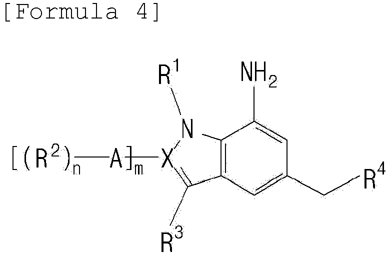 wherein, in Formulae above, n, m, X, A, R¹, R², R³, and R⁴ are the same as defined in claim 1.

4. The method of claim 3, wherein the primary reduction is performed by using, as a reducing agent, at least one selected from the group consisting of sodium borohydride (NaBH₄), boron trifluoride etherate (BF₃OEt₂), and lithium aluminum hydride (LAH).

5. The method of claim 3, wherein the secondary reduction is performed by using, as a reducing agent, at least one selected from the group consisting of Pd/C, NiBr₂, NiCl₂, ZnCl₂, ZrCl₄, I₂, BiCl₃, Cu, FeCl₃ -6H₂O, In (OTf₃), Ni, NiCl₂·6H₂O, SnCl₂, LiBH₄, LiCl, NaBF₄, and CoCl₂·6H₂O.

6. The method of claim 3, comprising a step for reacting the compound represented by Formula 4 above with ketone or aldehyde to prepare the compound represented by Formula 2 above.

## Patentansprüche

1. Verfahren zur Herstellung einer Verbindung, dargestellt durch nachstehende Formel 2, umfassend eine Verbindung, dargestellt durch nachstehende Formel 1, als Reaktionszwischenprodukt: 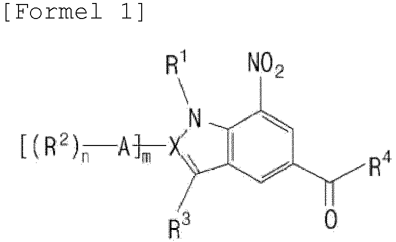 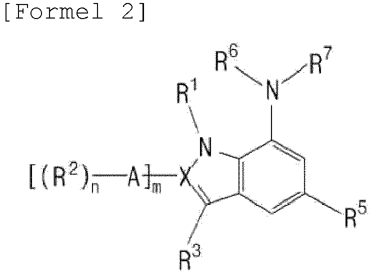
wobei in obigen Formeln,
n eine ganze Zahl von 1 bis 3 ist,
m 0 oder 1 ist,
A Phenyl darstellt, oder 5-gliedrigen Heteroaryl oder Heterocyclus darstellt, welche jeweils 1 bis 3 Heteroatome ausgewählt aus N-, O- und S-Atomen enthalten und gegebenenfalls mit R substituiert sind, wobei R Wasserstoff oder C₁-C₄-Alkyl, gegebenenfalls substituiert mit Hydroxy oder Amino, darstellt,
X C oder N darstellt, mit der Maßgabe, dass m 0 ist, wenn X N ist, und m 1 ist, wenn X C ist,
R¹ Wasserstoff, C₁-C₆-Alkyl oder -(CH₂)ᵣNR⁸R⁹ darstellt, wobei r eine ganze Zahl von 2 bis 5 ist, R⁸ und R⁹ jeweils unabhängig Wasserstoff oder C₁-C₃-Alkyl darstellen, mit der Maßgabe, dass R¹ Wasserstoff ist, wenn X N ist,
R² Wasserstoff, Halogen oder C₁-C₆-Alkoxy darstellt, oder -(CH₂)ₚCO₂R⁸, -(CH₂)ₚOR⁸, -(CH₂)ₚNR⁸R⁹, -NHR¹⁰, -N(H)S(O)₂R⁸, oder -NHC(O)R¹⁰ darstellt, oder -(CH₂)ₚ-Heterocyclus-R¹⁰ darstellt, in welchem die Heterocycluseinheit ein 5-6-gliedriger Ring ist, enthaltend ein oder zwei Heteroatome ausgewählt aus N-, O- und S-Atomen, wobei p eine ganze Zahl von 0 bis 3 ist, R⁸ und R⁹ wie oben definiert sind, und R¹⁰ Wasserstoff, Oxo, C₁-C₆-Alkylcarbonyl, C₁-C₆-Alkoxy oder C₁-C₆-Alkyl darstellt oder einen 5-6-gliedrigen Heterocyclus darstellt, enthaltend ein oder zwei Stickstoffatome als ein Heteroatom,
R³ Wasserstoff, Halogen, C₁-C₆-Alkyl oder Phenyl darstellt, oder -(CH₂)ₛ-Heterocyclus darstellt, in welchem der Heterocyclus ein 5-6-gliedriger Ring ist, enthaltend ein oder zwei Heteroatome ausgewählt aus N- und O-Atomen, wobei s eine ganze Zahl von 1 bis 3 ist, mit der Maßgabe, dass R³ Wasserstoff oder Phenyl ist, wenn X N ist,
R⁴ -YR¹¹ darstellt, wobei Y eine direkte Bindung ist oder -(CR⁸R⁹)_{g}- darstellt, wobei g eine ganze Zahl von 0 bis 3 ist, R⁸ und R⁹ die gleichen wie oben definiert sind, R¹¹ ausgewählt ist aus der Gruppe bestehend aus Wasserstoff, Halogen, C₁-C₆-Alkyl und -(CH₂)ₜB-R¹³, t eine ganze Zahl von 0 bis 3 ist, B einen 5-6-gliedrigen Heterocyclus darstellt, enthaltend ein oder zwei Heteroatome ausgewählt aus N-, O- und S-Atomen, oder C₆-C₁₀-Aryl darstellt, R¹³ Wasserstoff, Cyano, Halogen, Hydroxy, Oxo, Thiol, Carboxy oder Carboxy-C₁-C₆-alkyl darstellt, mit der Maßgabe, dass R⁴ Wasserstoff oder C₁-C₆-Alkyl darstellt, wenn X N ist,
R⁵ -Y'R¹¹ darstellt, wobei Y' eine direkte Bindung ist oder -(CR⁸R⁹)ₕY"- darstellt, wobei h eine ganze Zahl von 0 bis 3 ist, und R⁸, R⁹ und R¹¹ die gleichen wie oben definiert sind,
Y" ausgewählt ist aus der Gruppe bestehend aus -O-, -C(O)- und -C(O)O-, mit der Maßgabe, dass R⁵ Wasserstoff oder C₁-C₆-Alkyl darstellt, wenn X N ist,
R⁶ Wasserstoff, C₁-C₆-Alkyl, C₃-C₆-Cycloalkyl, Heterocyclus oder Heterocyclyl-C₁-C₆-alkyl darstellt, wobei der Heterocyclus ein 3-8-gliedriger Ring ist, enthaltend ein bis drei Heteroatome ausgewählt aus N- und O-Atomen, mit der Maßgabe, dass R⁶ Wasserstoff ist, wenn X N ist, und
R⁷ -(CR⁸R⁹)ᵤ-Z-D-W-R¹⁴ darstellt, wobei u eine ganze Zahl von 0 bis 3 ist, Z eine direkte Bindung darstellt oder ausgewählt ist aus der Gruppe bestehend aus -C(O)- und - C(O)O-, D eine direkte Bindung, C₄-C₆-Cycloalkyl oder ein 5-6-gliedriges Heteroaryl, enthaltend ein oder zwei N-Atome, darstellt, oder einen 5-6-gliedrigen Heterocyclus, enthaltend ein oder zwei Heteroatome ausgewählt aus N-, O- und S-Atomen, darstellt, W eine direkte Bindung oder -NR⁸-, -C(O)-, -C(O)O-, -C(O)NR¹²- oder -S(O)_{y}- darstellt, R¹² Wasserstoff, C₁-C₃-Alkyl oder C₆-C₁₀-Aryl darstellt, y eine ganze Zahl von 1 oder 2 ist, und R¹⁴ Wasserstoff, Hydroxy, C₁-C₆-Alkyl, einen 5-6-gliedrigen Heterocyclus, enthaltend ein bis drei Heteroatome ausgewählt aus N, O- und S-Atomen, oder C₆-C₁₀-Ar-C₁-C₆-Alkyl darstellt, mit der Maßgabe, dass, wenn X N ist, R⁷ C₄-C₆-Cycloalkyl oder einen 5-6-gliedrigen Heterocyclus, enthaltend ein oder zwei Heteroatome ausgewählt aus N-, O- und S-Atomen, darstellt,
wobei Alkyl, Alkoxy, Aryl, Cycloalkyl, Heterocyclus und Heteroaryl optional substituiert sein können und die Substituenten einer oder mehrere ausgewählt aus der Gruppe bestehend aus Hydroxy, C₁-C₆-Alkylamino, Di(C₁-C₆-alkyl)amino, Carboxy, C₁-C₆-Alkyl, C₁-C₆-Alkoxy, Carboxy-C₁-C₆-alkyl and Oxo sind.

2. Verfahren gemäß Anspruch 1, wobei die durch obige Formel 1 dargestellte Verbindung durch Amidierung der durch nachstehende Formel 3 dargestellte Verbindung hergestellt ist: 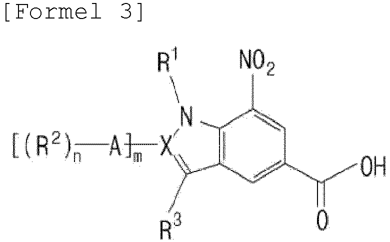 wobei in obiger Formel n, m, X, A, R¹, R² und R³ die gleichen wie in Anspruch 1 definiert sind.

3. Verfahren gemäß Anspruch 1, umfassend einen Schritt zur Durchführung einer primären Reduktion und einer sekundären Reduktion der durch obige Formel 1 dargestellten Verbindung, um eine durch nachstehende Formel 4 dargestellte Verbindung herzustellen: 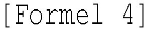 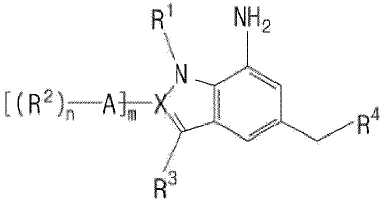 wobei in obigen Formeln n, m, X, A, R¹, R², R³ and R⁴ die gleichen wie in Anspruch 1 definiert sind.

4. Verfahren gemäß Anspruch 3, wobei die primäre Reduktion unter Verwendung von mindestens einem, als ein Reduktionsmittel, ausgewählt aus der Gruppe bestehend aus Natriumborhydrid (NaBH₄), Bortrifluoridetherat (BF₃OEt₂) und Lithiumaluminiumhydrid (LAH) durchgeführt wird.

5. Verfahren gemäß Anspruch 3, wobei die sekundäre Reduktion unter Verwendung von mindestens einem, als ein Reduktionsmittel, ausgewählt aus der Gruppe bestehend aus Pd/C, NiBr₂, NiCl₂, ZnCl₂, ZrCl₄, I₂, BiCl₃, Cu, FeCl₃·6H₂O, In(OTf₃), Ni, NiCl₂·6H₂O, SnCl₂, LiBH₄, LiCl, NaBF₄ und CoCl₂·6H₂O durchgeführt wird.

6. Verfahren gemäß Anspruch 3, umfassend einen Schritt zur Reaktion der durch obige Formel 4 dargestellten Verbindung mit Keton oder Aldehyd zur Herstellung der durch obige Formel 2 dargestellten Verbindung.

## Revendications

1. Procédé de préparation d'un composé représenté par la formule 2 ci-dessous en comprenant un composé représenté par la formule 1 ci-dessous en tant qu'intermédiaire réactionnel : 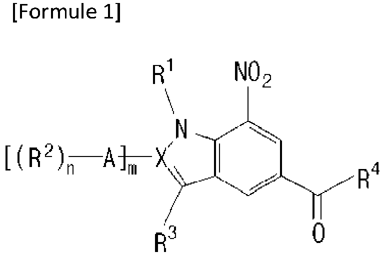 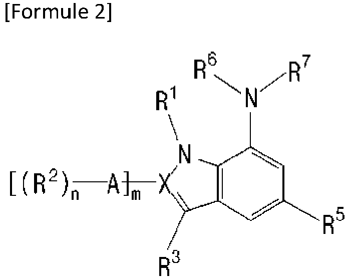
dans lequel, dans les formules ci-dessus,
n est un nombre entier de 1 à 3,
m est 0 ou 1,
A représente un phényle, ou représente un hétéroaryle ou hétérocycle à 5 chaînons contenant chacun 1 à 3 hétéroatomes sélectionnés parmi les atomes N, O et S et est facultativement substitué par R, dans lequel R représente un hydrogène ou un alkyle en C₁-C₄ facultativement substitué par un hydroxy ou un amino,
X représente C ou N, à condition que m soit 0 quand X est N et m soit 1 quand X est C,
R¹ représente un hydrogène, un alkyle en C₁-C₆ ou -(CH₂)ᵣNR⁸R⁹, dans lequel r est un nombre entier de 2 à 5, R⁸ et R⁹ représentent chacun indépendamment un hydrogène ou un alkyle en C1-C3, à condition que R¹ soit un hydrogène quand X est N,
R² représente un hydrogène, un halogène ou un alcoxy en C₁-C₆, ou représente (CH₂)_{P}CO₂R⁸, -(CH₂)_{P}OR⁸, - (CH₂)_{P}NR⁸R⁹, -NHR¹⁰, -N(H)S(O)₂R⁸, ou -NHC(O)R¹⁰, ou représente un - (CH₂)ₚ-hétérocycle-R¹⁰ dans lequel le fragment hétérocycle est un cycle à 5-6 chaînons contenant un ou deux hétéroatomes sélectionnés parmi les atomes N, O, et S, dans lequel p est un nombre entier de 0 à 3, R⁸ et R⁹ sont tels que précédemment définis, et R¹⁰ représente un hydrogène, un oxo, un alkylcarbonyle en C₁-C₆, un alcoxy en C₁-C₆ ou un alkyle en C₁-C₆, ou représente un hétérocycle à 5-6 chaînons contenant un ou deux atomes d'azote en tant qu'hétéroatome,
R³ représente un hydrogène, un halogène, un alkyle en C₁-C₆ ou un phényle, ou représente un -(CH₂)ₛ-hétérocycle dans lequel l'hétérocycle est un cycle à 5-6 chaînons contenant un ou deux hétéroatomes sélectionnés parmi les atomes N et O, dans lequel s est un nombre entier de 1 à 3, à condition que R³ soit un hydrogène ou un phényle quand X est N,
R⁴ représente -YR¹¹, dans lequel Y est une liaison directe ou représente -(CR⁸R⁹)_{g}-, dans lequel g est un nombre entier de 0 à 3, R⁸ et R⁹ sont tels que précédemment définis, R¹¹ est sélectionné dans le groupe consistant en un hydrogène, un halogène, un alkyle en C₁-C₆ et -(CH₂)ₜB-R¹³, t est un nombre entier de 0 à 3, B représente un hétérocycle à 5-6 chaînons contenant un ou deux hétéroatomes sélectionnés parmi les atomes N, O, et S, ou représente un aryle en ₆-C₁₀, R¹³ représente un hydrogène, un cyano, un halogène, un hydroxy, un oxo, un thiol, un carboxy ou un carboxy-alkyle en C₁-C₆, à condition que R4 représente un hydrogène ou un alkyle en C₁-C₆ quand X est N,
R⁵ représente -Y'R¹¹, dans lequel Y' est une liaison directe ou représente -(CR⁸R⁹)ₕY"-, dans lequel h est un nombre entier de 0 à 3, et R⁸, R⁹ et R¹¹ sont tels que précédemment définis,
Y" est sélectionné dans le groupe consistant en -O-, -C(O)-, et -C(O)O-, à condition que R⁵ représente un hydrogène ou un alkyle en C₁-C₆ quand X est N,
R⁶ représente un hydrogène, un alkyle en C₁-C₆, un cycloalkyle en C₃-C₆, un hétérocycle ou un hétérocyclyle-alkyle en C₁-C₆, dans lequel l'hétérocycle est un cycle à 3-8 chaînons contenant un à trois hétéroatomes sélectionnés parmi les atomes N et O, à condition que R6 soit un hydrogène quand X est N, et
R⁷ représente -(CR⁸R⁹)ᵤ-Z-D-W-R¹⁴, dans lequel u est un nombre entier de 0 à 3, Z représente une liaison directe ou est sélectionné dans le groupe consistant en -C(O)- et - C(O)O-, D représente une liaison directe, un cycloalkyle en C₄-C₆, ou un hétéroaryle à 5-6 chaînons contenant un ou deux atomes N, ou représente un hétérocycle à 5-6 chaînons contenant un ou deux hétéroatomes sélectionnés parmi les atomes N, O, et S, W représente une liaison directe, ou -NR⁸- , -C(O)-, -C(O)O-, -C(O)NR¹²- ou -S(O)_{y}-, R¹² représente un hydrogène, un alkyle en C₁-C₃- ou un aryle en C₆-C₁₀, y est un nombre entier égal à 1 ou 2, et R¹⁴ représente un hydrogène, un hydroxy, un alkyle en C₁-C₆, un hétérocycle à 5-6 chaînons contenant un à trois hétéroatomes sélectionnés parmi les atomes N, O, et S, ou un Ar en C₆-C₁₀-alkyle en C₁-C₆, à condition que quand X est N, R⁷ représente un cycloalkyle en C₄-C₆, ou un hétérocycle à 5-6 chaînons contenant un ou deux hétéroatomes sélectionnés parmi les atomes N, O, et S,
dans lequel les alkyle, alcoxy, aryle, cycloalkyle, hétérocycle et hétéroaryle peuvent être facultativement substitués, et les substituants sont un ou plusieurs sélectionnés dans le groupe consistant en un hydroxy, un (alkyle en C₁-C₆)amino, un di(alkyle en C₁-C₆)amino, un carboxy, un alkyle en C₁-C₆, un alcoxy en C₁-C₆, un carboxy-alkyle en C₁-C₆ et un oxo.

2. Procédé selon la revendication 1, dans lequel le composé représenté par la formule 1 ci-dessus est préparé par l'amidation du composé représenté par la formule 3 ci-dessous : 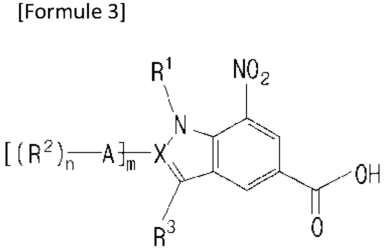 dans lequel, dans la formule ci-dessus, n, m, X, A, R¹, R², et R³ sont tels que définis dans la revendication 1.

3. Procédé selon la revendication 1, comprenant une étape de mise en oeuvre d'une réduction primaire et d'une réduction secondaire du composé représenté par la formule 1 ci-dessus pour préparer un composé
représenté par la formule 4 ci-dessous : 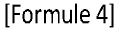 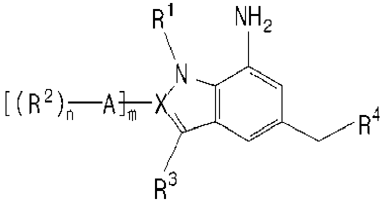 dans lequel, dans la formule ci-dessus, n, m, X, A, R¹, R², R³, et R⁴ sont tels que définis dans la revendication 1.

4. Procédé selon la revendication 3, dans lequel la réduction primaire est effectuée en utilisant, comme agent réducteur, au moins un sélectionné dans le groupe consistant en le borohydrure de sodium (NaBH₄), l'éthérate de trifluorure de bore (BF₃OEt₂) et l'hydrure de lithium et d'aluminium (LAH).

5. Procédé selon la revendication 3, dans lequel la réduction secondaire est effectuée en utilisant, comme agent réducteur, au moins un sélectionné dans le groupe consistant en Pd/C, NiBr₂, NiCl₂, ZnCl₂, ZrCl₄, I₂, BiCl₃, Cu, FeCl₃-6H₂O, In(OTf₃), Ni, NiCl₂-6H₂O, SnCl₂, LiBH₄, LiCI, NaBF₄ et CoCl₂-6H₂O.

6. Procédé selon la revendication 3, comprenant une étape de réaction du composé représenté par la formule 4 ci-dessus avec une cétone ou un aldéhyde pour préparer le composé représenté par la formule 2 ci-dessus.
